# EUROPEAN PATENT APPLICATION

(11) **EP 2 463 293 A1**
(43) Date of publication of application: **13.06.2012**
(21) Application number: 09847968.6
(22) Date of filing: 06.08.2009
(51) Int. Cl.: C07K 7/56, C07K 1/107, A61K 38/12, A61P 31/10

(54) **AZACYCLOHEXAPEPTIDE OR ITS PHARMACEUTICAL ACCEPTABLE SALT, PREPARING METHOD AND USE THEREOF**

(71) Applicant: Shanghai Techwell Biopharmaceutical Co., Ltd, Shanghai 201108 (CN)
(72) Inventor: XU, Tianhui, Shanghai 201108 (CN); FANG, Tao, Shanghai 201108 (CN); ZHUO, Zhonghao, Shanghai 201108 (CN); ZHENG, Yunman, Shanghai 201108 (CN); JI, Xiaoming, Shanghai 201108 (CN)
(74) Representative: Mattsson, Niklas
(86) International application number: PCT/CN2009/073112
(87) International publication number: WO 2011/014990

(57) **Abstract**

The present invention disclosed a novel azacyclohexapeptide or pharmaceutically acceptable salts, preparation methods and uses thereof. The structure of the azacyclohexapeptide is represented by the following formula 4:

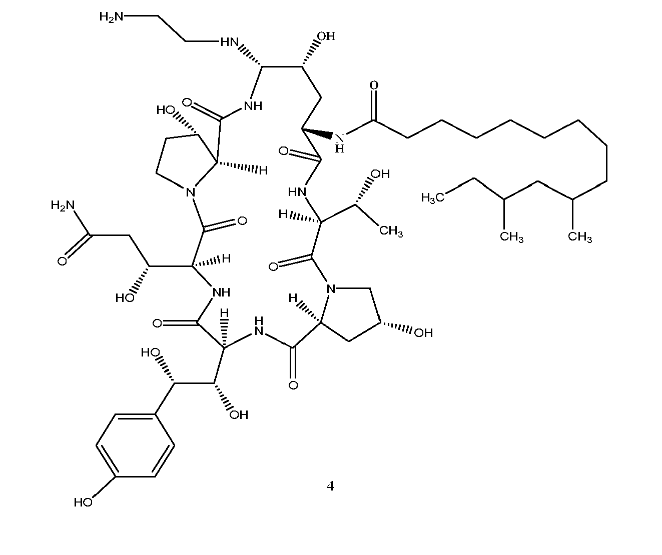

## Description

### FIELD OF THE INVENTION

The present invention relates to the art of organic compounds, especially, relates to an azacyclohexapeptide or pharmaceutically acceptable salts and preparation methods and uses thereof.

### BACKGROUND OF THE INVENTION

In 1974 , it was discovered that echinocandin compounds possess favourable antibacterial activity. Since then, pharmacological activity of many semisynthetic echinocandin compounds have been studied. In 2001 , caspofungin was approved by FDA of the United States, which represents the landmark for antifungal medicaments. Caspofungin is a low-toxic agent with unique action site and broad spectrum, represented by the following formula 1 :

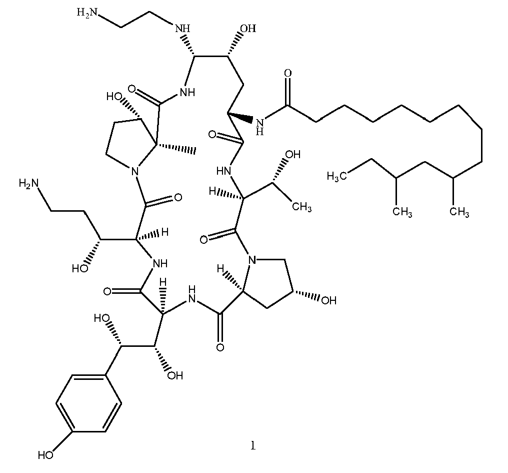

In the prior art, multiple reaction steps are necessary for producing caspofungin , and the stereoselectivity or yield of the synthesized compounds is poor, thus unsuitable for mass production. In US patent No.5,378,804, caspofungin (the compound 1) was produced in 5 steps , however, the total yield was merely 6.3%.

Therefore, there is an urgent need in the art for a novel preparation method of caspofungin.

### SUMMARY OF THE INVENTION

The object of the present invention is to provide an azacyclohexapeptide or pharmaceutically acceptable salts thereof.

Another object of the present invention is to provide preparation methods for the azacyclohexapeptide or pharmaceutically acceptable salts thereof.

A further object of the present invention is to provide uses of the azacyclohexapeptide or pharmaceutically acceptable salts thereof.

In the first aspect, the invention provides an azacyclohexapeptide represented by the following formula 4 or pharmaceutically acceptable salts thereof :

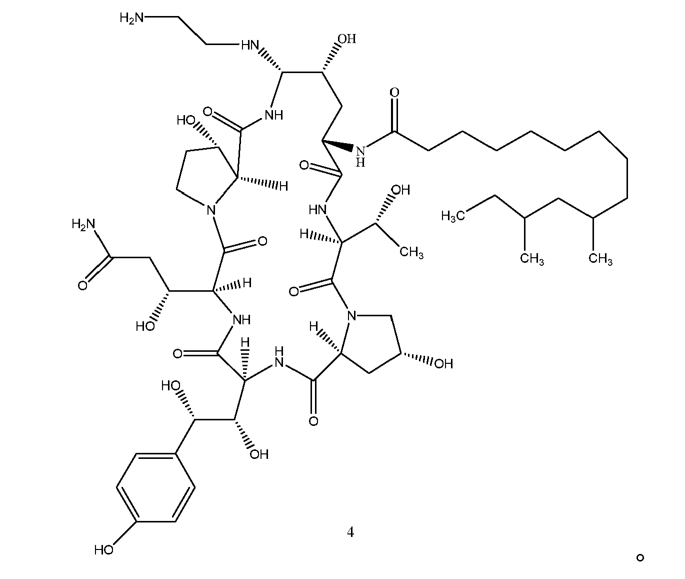

In one preferred embodiment, the method comprises the following steps:
( a ) mixing the compound of formula 2 with a compound containing strong leaving-group to obtain the compound of formula 3; and
( b ) mixing the compound of formula 3 with ethylenediamine to obtain the azacyclohexapeptide or pharmaceutically acceptable salts thereof of claim 1 ; wherein the compound containing strong leaving-group is the sulfhydryl-substituted aromatic ring compound represented by R-SH, wherein R is selected from phenyl, 4-methoxyphenyl, methylimidazolyl, or benzimidazolyl;

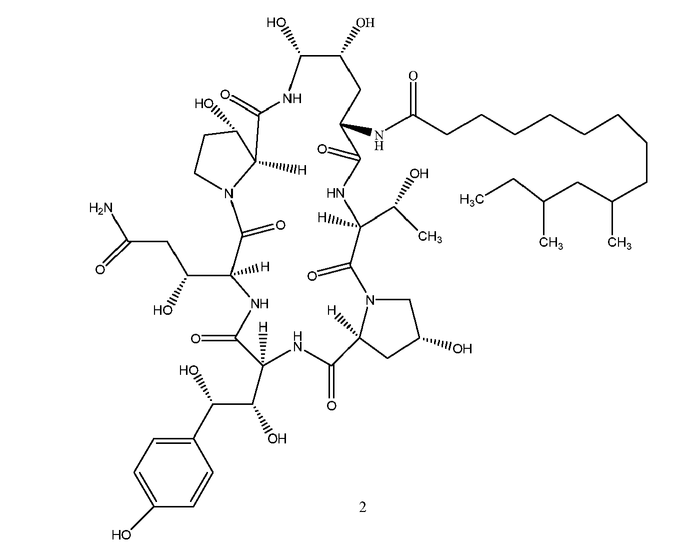

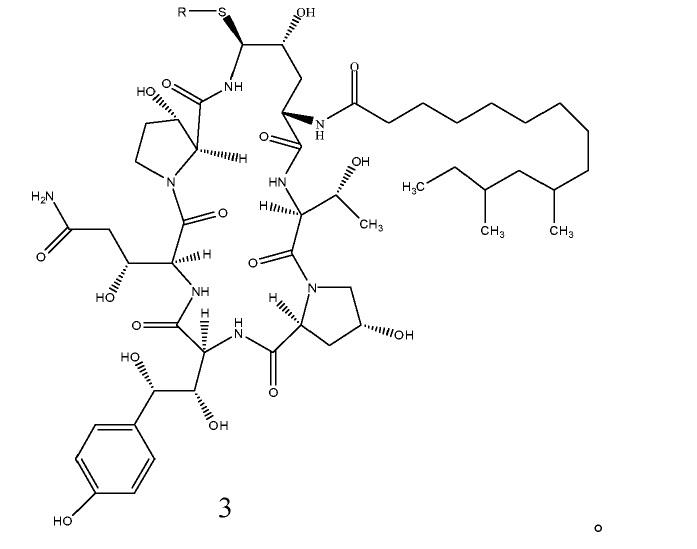

In another preferred embodiment, step (a) comprises mixing the compound of formula 2 with the compound containing strong leaving-group dissolved in an acid solution, wherein the acid is selected from p-toluene sulfonic acid, methane-sulfonic acid, camphorsulfonic acid, trifluoromethanesulfonic acid or trifluoroacetic acid.

In another preferred embodiment, the temperature for performing the mixing in step (a) is in a range of -50°C to 40°C; preferably at -15°C.

In another preferred embodiment, step (b) comprises mixing the compound of formula 3 with ethylenediamine dissolved in the following solvents: water, methanol, ethanol, aqueous alcohol, tetrahydrofuran, isopropanol, trifluoroethanol, acetonitrile, or dichloromethane.

In another preferred embodiment, the aqueous alcohol is selected from aqueous methanol or aqueous ethanol.

In another preferred embodiment, the temperature for performing the mixing in step (b) is in a range of -10°C to 40°C; preferably at 25°C.

In the second aspect, the invention provides the use of the azacyclohexapeptide or pharmaceutically acceptable salts thereof according to the invention in the manufacture of a medicament for preventing or treating diseases caused by fungous infection.

In the third aspect, the invention provides the preparation method for the compound of formula 1, comprising the following steps:
( i ) mixing the compound of formula 4 with a hydroxyl protecting agent to obtain the compound of formula 4 with hydroxy being protected; and
( ii ) mixing the compound of formula 4 with hydroxy being protected with borane complex to obtain the compound of formula 1; 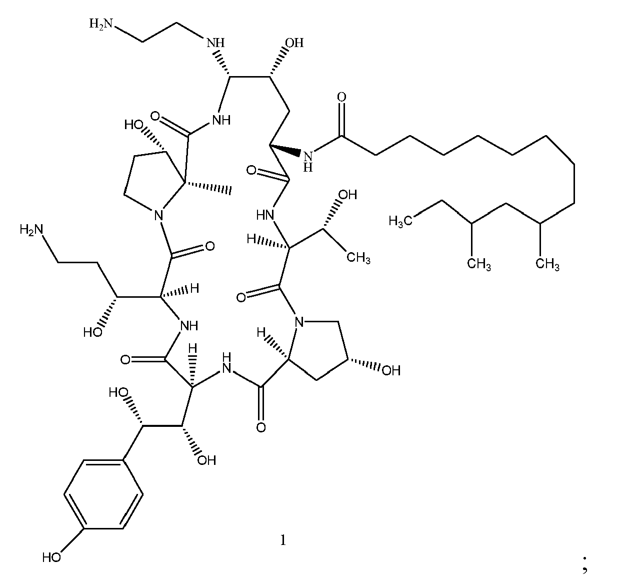
wherein the hydroxyl-protecting agent is selected from boric acid protective agents or silane agents;
wherein the borane complex is selected from the complex of methyl borane and tetrahydrofuran, methyl borane and dimethyl sulfide, methyl borane and dibenzyl sulfide, methyl borane and diphenyl sulfide, methyl borane and 1,4-oxathiane, or the complex of BH₂CI and dimethyl sulfide; preferably, the complex of methyl borane and tetrahydrofuran, or methyl borane and dimethyl sulfide.

In another preferred embodiment, the temperature for performing the mixing in step ( ii ) is in a range of -20°C - 40°C; preferably 0°C - 10°C.

In another preferred embodiment, the method comprises the following steps:
( 1 ) mixing the compound of formula 2 with the compound containing strong leaving-group to obtain the compound of formula 3 ;
( 2 ) mixing the compound of formula 3 with ethylenediamine to obtain the azacyclohexapeptide or pharmaceutically acceptable salts thereof according to the invention ; and
( 3 ) mixing the compound of formula 4 with borane complex to obtain the compound of formula 1,
wherein the compound containing the strong leaving-group is the sulfhydryl-substituted aromatic ring compound represented by R-SH, wherein R is selected from phenyl, 4-methoxyphenyl, methylimidazolyl, or benzimidazolyl; wherein the borane complex is selected from the complex of methyl borane and tetrahydrofuran, methyl borane and dimethyl sulfide, methyl borane and dibenzyl sulfide, methyl borane and diphenyl sulfide, methyl borane and 1,4-oxathiane, or the complex of BH₂CI and dimethyl sulfide.

Therefore, the invention provides a novel preparation method for caspofungin.

### SPECIFIC EMBODIMENTS

The inventor has firstly discovered a novel compound, *i.e*., the compound of formula 4, and a convenient preparation method for the compound of formula 4. The inventor has discovered that the compound of formula 1, *i.e.,* caspofungin could be conveniently obtained by using the compound of formula 4 as intermediate through reduction reaction. Additionally, the inventor has discovered that the compound of formula 4 itself could prevent or treat infectious deseases as well. Therefore, the invention is made based on these discoveries.

As used herein, the chemical formula or name should include all the optical isomers and stereoisomer as well as racemic mixture consisting of these isomers.

### COMPOUNDS

The present invention provides a compound of formula 4 and pharmaceutically acceptable salts thereof,

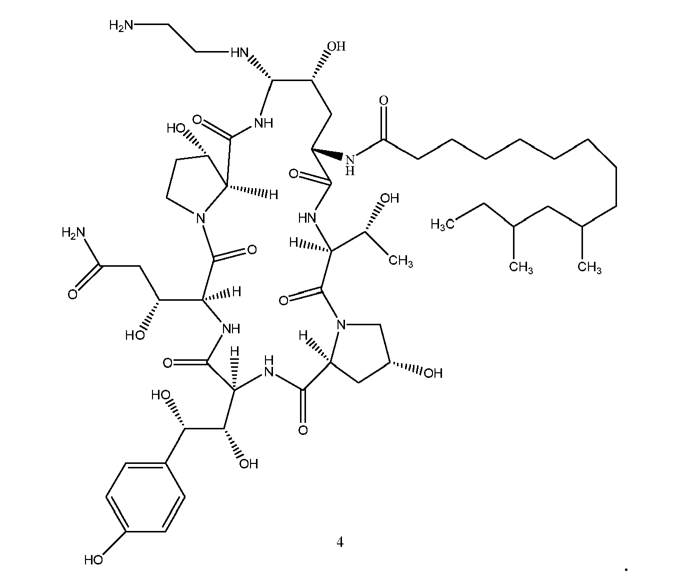

The compounds provided by the invention are generally obtained as mixtures of stereoisomeric forms in which one form usually predominates. Conditions may be adjusted by means within the normal skill of the skilled artisan to obtain predominantly the desired isomer. The compound with preferred stereoisomeric form as the "normal form" is the compound, in which the group at "C-5-orn" position is below the plane of the position. The designation "epi" has been employed for those compounds in which the group at "C-5-orn" position is above the plane. The "C-5-orn" position is the fifth carbon of 4-hydroxyornithine moiety.

The pharmaceutically acceptable salts provided by the invention as acid addition salts are those derived from the following acids: hydrochloric acid, hydrobromic acid, phosphoric acid, sulphuric acid, maleic acid, citric acid, acetic acid, tartaric acid, succinic acid, oxalic acid, malic acid, glutamic acid, etc., and includ other acids related to the pharmaceutically acceptable salts listed in the Juornal of Pharmaceutical Sciences, 66 : 2 ( 1977 ) .

### PREPARATION METHOD

The present invention provides a praparation method for the compound of formula 4, comprising the following steps:

The first step, reacting the compound of formula 2 with a compound containing strong leaving group to obtain the compound of formula 3 ;

The second step, reacting the compound of formula 3 with ethylenediamne to form the compound of formula 4.

Further, reacting the resulted compound of formula 4 with a reducing agent to obtain the compound of formula 1.

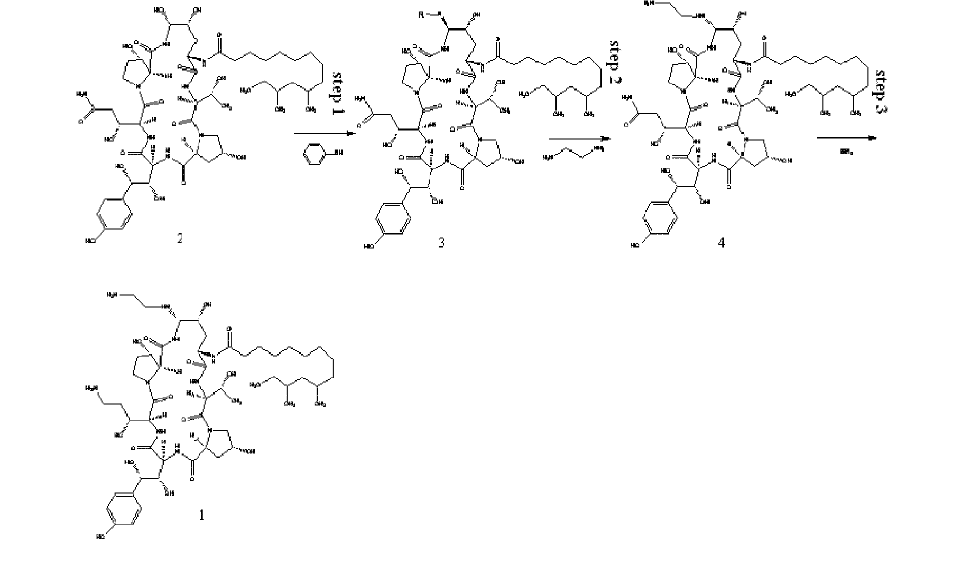

In the method according to the invention, the compound of formula 2 can be prepared by the method well known in the art, such as but not limited to the method described in US Patent No. 5,021,341 published in June 4, 1991: cultivation of Zalerion arboricola ATCC 20868 in nutrient medium rich in mannitol as prime carbon source.

The compound containing strong leaving-group in the invention is the sulfhydryl-substituted aromatic ring compound represented by R-SH, wherein R is selected from phenyl, 4-methoxyphenyl, methylimidazolyl, or benzimidazolyl, preferably thiophenol.

The catalyst used in the first step can be a medium-strength acid, such as but not limited to toluene sulfonic acid, methane-sulfonic acid, camphorsulfonic acid, trifluoromethanesulfonic acid or trifluoroacetic acid (TFA).

In one embodiment according to the invention, in the first step, the compound of formula 2 may be reacted with thiophenol dissolved in acetonitrile and TFA to obtain the intermediate containing phenyl sulfide , *i.e*. the compound of formula 3. The reaction liquid can be neutralized with sodium acetate solution to obtain a stable amorphous solid intermediate .

The substitution rate and the formation of the undesirable diphenyl sulfide (the compound of formula 5) in the cyclic peptide fragment rich in tyrosine are dependent on the intensity and amount of acids used. Preferably, the solvent mixture of acetonitrile and TFA comprises 5-25v/v% TFA, more preferably, 7-15% TFA, based on the total volume of the mixture.

According to a preferred embodiment of the invention, in the first step, the phenyl boric acid can be added to protect the o-dihydroxyl group in the fragment rich in tyrosine to obtain the phenyl borate intermediate (the compound of formula 6), thus significantly reducing the formation of impurity *i.e*. diphenyl sulfide, and lowering the reaction temperature. More preferably, the stronger acid, such as trifluoromethanesulfonic acid can be used as catalyst, when phenyl boric acid is used to protect the o-hydroxyl .

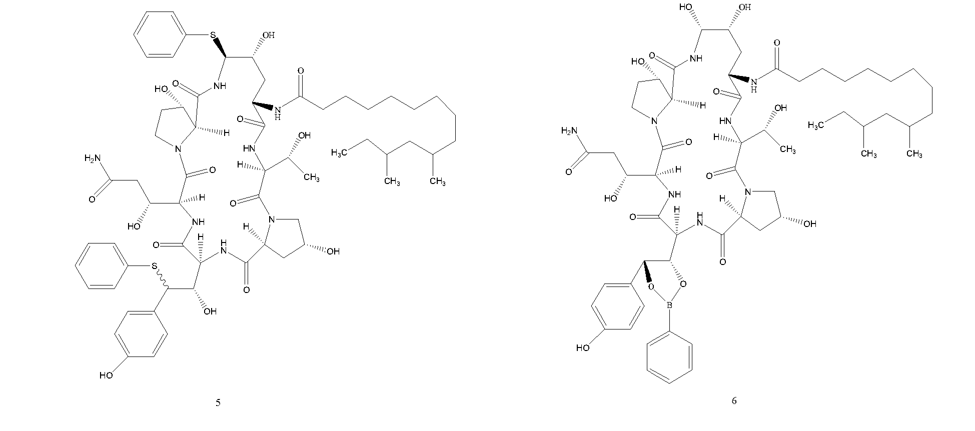

According to a preferred embodiment of the invention, the amount of thiophenol used in the first step is 3-5 N; more preferably, 3 N thiophenol, 2 N phenyl boric acid and 3 N trifluoromethanesulfonic acid dissolved in acetonitrile were used at -15°C to form the sulfides, with 80-90% yield upon crystallization.

According to an embodiment of the invention, in the second step, the compound of formula 3 was reacted with 1,2-ethylenediamine in polar solvent to obtain the compound of formula 4.

Preferably, the reaction may be performed at -10°C to 40°C for 0.5-24 hr; more preferably, for 1.5 hr at ambient temperature.

Preferably, the polar solvent is selected from water, methanol, ethanol, isopropanol, aqueous alcohol, tetrahydrofuran, trifluoroethanol, dichloromethane or acetonitrile. The aqueous alcohol is selected from aqueous methanol or aqueous ethanol.

In one embodiment according to the invention, pH is adjusted to 5-6 with acetic acid upon the completion of the second step, then the mixture diluted by water was purified through column chromatography, and the dried solid intermediate (*i.e*., the compound of formula 4) was obtained through concentration, or crystallization. In a preferred embodiment according to the invention, said column chromatography is performed on a reverse phase column eluted with aqueous organic solvent, which may be selected from methanol, acetonitrile, isopropanol and the like.

In the step of reducing amide(the compound of formula 4) to amine, the reducing agent may be selected from methyl borane complex, metal boride dissolved in THF or other suitable solvents, titanium or zirconium boride, or methyl borane complex and ammonia, dimethylamine, pyridine or piperazine; with methyl borane complex, metal boride dissolved in THF or other suitable solvents being preferred. The methyl borane complex may be selected from the complex of methyl borane and tetrahydrofuran (THF), dimethyl sulfide, diphenyl sulfide, dibenzyl sulfide, 1,4-oxathiane or BH₂CI and dimethyl sulfide; the metal boride dissolved in THF or other suitable solvents is selected from the complex of ZrC1₄/NaBH₄ or TiCl₄/NaBH₄. The unreduced amide can be removed through reverse phase chromatography.

In a preferred embodiment according to the invention, firstly, the o-dihydroxyl group in the fragment rich in tyrosine can be protected, and then the amide was reduced by a reducing reagent to obtain the compound of formula 1. The o-dihydroxyl group in the fragment may be protected by phenyl boric acid and the remaining hydroxyl group may be protected by N,O-di(trimethylsilane)trifluoroacetamide (BSTFA ) , forming a homogeneous reaction liquid, thus greatly improving the conversion ratio. Preferably, reacting the compound 4 with 1.1-2 N pheny boric acid in tetrahydrofuran solution at 25-80°C; adding 3-7 N BSTFA and reacting at 0-80°C to form homogenous reaction liquid; then adding borane and reacting at -30°Cto 20°C, thus forming compound of formula 1;and then the pure compound of formula 1 was obtained through column chromatography and crystallization.

Following the addition of BSTFA, the reaction may be refluxed for 20-40 min to form a homogeneous liquid, allowing pheny boric acid reacting with the compound of formula 4 sufficiently.

The invention also embraces acid addition salts. The compound of formula 4 in the normal course of isolation is obtained asan acid addition salts. Generally, it is as a trifluoroacetic acid salt. Thus slat thus obtained may be dissolved in water and pass through an anion exchange column. The eluate containing the desired salt may be concentrated to recover the salts as a solid product.

### USE

A important use for the compound of formula 4 provided by the invention is that it can be used as a intermediate to obtain caspofungin *i.e*. the compound of formula 1, which is the step of reducing amide (the compound of formula 4) to amine as described above.

Additionally, the compound of formula 4 itself can effectively treat fungous infection, treat or prevent the infectious diseases caused by Candida or Aspergillus, or can be used to prepare the therapeutic or preventive medicament for infectious diseases.

Therefore, the invention also provides a pharmaceutical composition comprising the compound of formula 4 and pharmaceutically acceptable carriers.

As used herein, the term "effective amount"refers to an amount which is functional or active in human and/or animal and acceptable for human and /or animal.

As used herein, the term "pharmaceutically acceptable carrier" refers to the carrier which can be used to administer therapeutical agents, including various excipients and diluents. The term refers to some vehicles which are not necessary active components and won't produce undue toxicity upon administration. The appropriate carriers are well known to the skilled in the art. Summary about pharmaceutically acceptable carrier can be found in Remington's Pharmaceutical Sciences(Mack Pub. Co. , N.J. 1991). The pharmaceutically acceptable carriers in the composition include liquid, such as water, saline, glycerol and ethanol. In addition, some auxiliary agents, such as disintegrant, moistening agent, emulsifier, pH buffer and the like, may be present in the composition.

The pharmaceutical compositions described herein can be made into various dosage forms based on specific administration routes, such as oral, spray, rectal, intranasal, buccal, topical, and parenteral administration, such as subcutaneous, intravenous, intramuscular, intraperitoneal, intrathecal, intraventricular, intrasternal, intracalvarium injection or infusion, or administration by a explantation reservoir.

All of the features disclosed in the present invention may be combined in any combination. An alternative feature serving the same, equivalent, or similar purpose may replace each feature disclosed in this specification. Thus, unless expressly stated otherwise, each feature disclosed is only an example of a generic serious of equivalent or similar features.

The advantages of the present invention reside in:
1. A novel azacyclohexapeptide or pharmaceutically acceptable salts thereof is provided by the invention.
2. The advantages of the preparation method provided by the invention, such as simple reaction steps, mild reaction condition, convenient work-up and high purity of products, to a great extent, reduce the requirement of manipulation and equipments as well as the cost.
3. Ecahinocandin (the compound 2) obtained by fermentation is used in the preparation method of the invention, and the synthesized solid intermediates are stable, therefore, it is favorable to the quality control of the intermediates and final product, as well as mass production.
4. The preparation method for the novel azacyclohexapeptide comprises merely 3 steps, the intermediates are stable, the yield is high and the product can be synthesized simply.

The invention is further illustrated in conjunction with the following examples. It is appreciated that these examples are onlt intended to illustrate the invention, but not to limit the scope of the invention.For the experimental methods in the following examples, they are performed under routine conditions, or as instructed by the manufacturers. Unless otherwise noted, all percentages, ratios, rates or parts are by weight.

The unit of w/v in the present invention is well known to a skilled person in the art, for example, the weight of solute in a 100 ml solution.

Unless defined otherwise, all technical and scientific terms used herein have the same meaning as commonly understood by the skilled in the art to which the invention belongs. In addition, any methods and materials similar or equivalent to those described herein can be used in the method of the present invention. The preferred embodiments and materials described herein are merely provided for illustration.

### Example 1: Preparation of compound 7 from compound 2

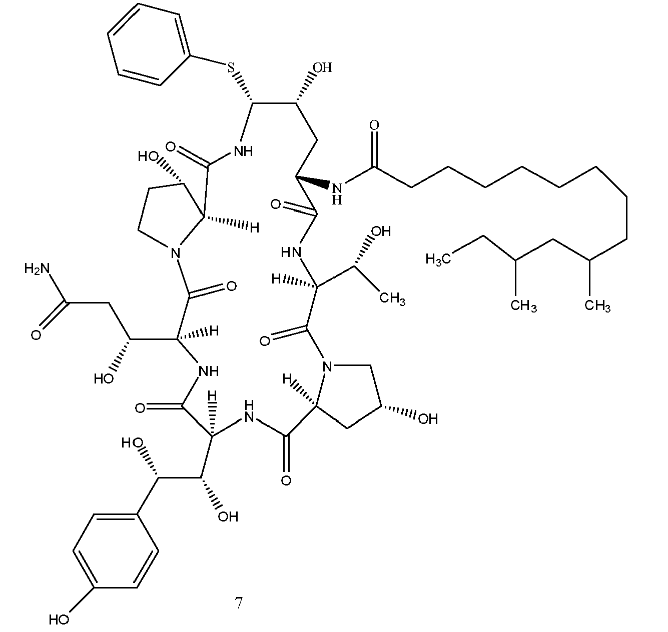

Compound 2( 100 g ,94.0 mmol ,ahhydrous and solvent free basis ), phenyl boric acid (22.9 g ,188 mmol )and thiophenol( 29.0 ml ,282 mmol ) were added into 3 L acetonitrile. The suspension was cooled to -15°C, and then triflioromethanesulfonic acid( 24.9 ml ,282 mmol )was added. The reaction was maintained at -15°C for 2.5 h. All the manipulations were performed under N₂. After the reaction was completed, sodium acetate solution ( 333 ml , 282 mol) was added, and a lot of precipitate formed. The suspension was warmed to 17°C, agitated for 2 hr, and then cooled to 0°C. The precipitate was filtered, washed with 1:9 ( v/v ) water/acetonitrile and dried to obtain compound 7 ( 93.4 g , 93.4% ).
MS(ESI) 1157.6 (M+H⁺ ) , 1179.6 (M+Na⁺ ) ;
1H NMR (500 MHz, CD30D)δ 7.56-7.55(om, 2H), 7.28-7.22 (om, 3H), 7.13 (m, 2H), 6.76-6.74 (m, 2H), 5.58 (d, 1H), 5.05 (d, 1H), 4.94(d, 1H), 4.57 (dd, 1H), 4.42-4.26 (om, 9H), 3.88 (om, 3H), 3.70 (om, 2H), 2.76 (dd, 1H), 2.45 (dd, 1H), 2.40 (om, 1H), 2.14-2.11 (om, 6H), 1.99 (m, 1H), 1.55(m, 2H), 1.32-1.20 (om, 15H), 1.10 (d, 3H), 1.10-1.08 (om, 2H), 0.91 (t, 1H), 0.87-0.86 (t, 3H), 0.84 (d, 3H), 0.83 (d, 3H);
13C NMR (125 MHz, CD3OD) 177.14, 175.9, 174.5, 173.7, 172.7, 172.0, 169.1, 158.7, 134.9, 133.2, 130.2, 130.0, 129.0, 116.5, 77.4, 76.0, 74.7, 71.6, 70.9, 70.7, 69.8, 68.4, 62.8, 61.7, 58.8, 57.3, 56.2, 55.9, 51.3, 49.8, 49.6, 49.4, 49.3, 49.1, 48.9, 48.7, 47.6, 47.0, 46.1, 40.0, 38.7, 38.3, 37.1, 36.3, 34.9, 33.1, 31.49, 31.45, 30.99, 30.94, 30.7, 30.6, 28.3, 27.4, 21.0, 20.5, 19.8

### Example 2: Preparation of compound 8 from compound 2

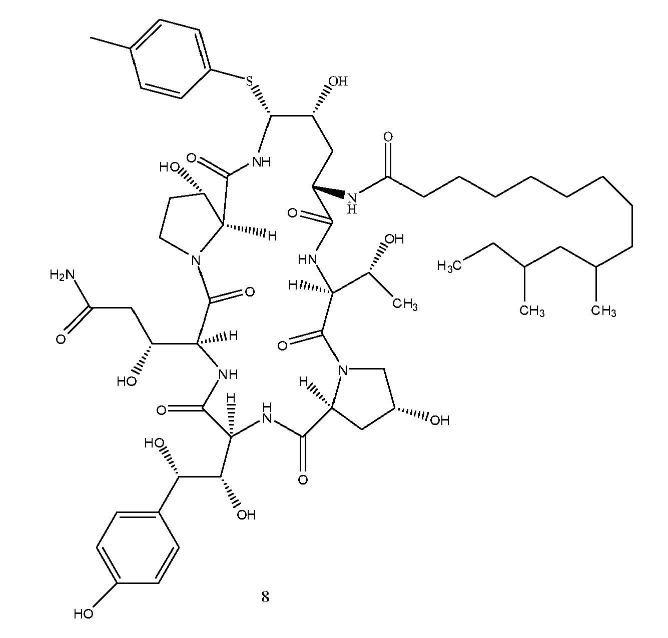

Compound 2 ( 10.0 g , 9.4 mmol, dried ) , phenyl boric acid (2.3 g , 18.8 mmol ) and p-methylthiophenol ( 3.56 g , 28.6 mmol ) were added into 3 L acetonitrile. The suspension was cooled to -15°C, and then triflioromethanesulfonic acid ( 2.49 ml , 28.2 mmol ) was added. The reaction was maintained at -15°C for 2.5 h. All the manipulations were performed under N₂. After the reaction was completed, sodium acetate solution ( 33.3 ml , 28.2 mol ) was added, and a lot of precipitate formed. The suspension was warmed to 17°C, agitated for 2 hr, and then cooled to 0°C. The precipitate was filtered, washed with 1:9 ( v/v ) water/acetonitrile and dried to obtain compound 5 ( 9.0 g,90%).

Under N₂ protection, the Compound 5 (9.0 g , 7.7 mmol) dissolved in 36 ml methanol was cooled to -10°C, ethylenediamine (36 ml, 537.8 mmol) was slowly added dropwise, and the temperature should be controlled not to exceed 2°C. The reaction was maintained at 30°C overnight, and then acetic acid was added to quench the reaction. A great amount of water was added, and then pH was adjusted to 5-6. Compound 4 (8.2 g, 91%) was obtained through concentration under vacuum and crystallization .
Ms(ESl) 1171.6 (M+H⁺)

### Example 3: Preparation of compound 4 from compound 7

Compound 7 (9.0 g, 7.7 mmol) dissolved in 37 ml methanol was cooled to -10°C, ethylenediamine (38.5 ml, 0.577 mol) was slowly added dropwise, and the temperature should be controlled not to exceed 2°C. The reaction was maintained at -10°C-0°C overnight, and then acetic acid was added to quench the reaction. pH was adjusted to 5-6, and the reaction mixture was purified using reverse-phase chromatography column (C18) (eluted with 10% - 60% acetonitrile/water gradient solution). Suitable eluate was collected, and lyophilized to obtain compound 4 (7.2 g, 80%).
MS(ESI) 1107.6(M+H⁺)

### Example 4: Preparation of compound 4 from compound 7

Compound 7 (3.0 g, 2.59 mmol) dissolved in 13 ml water was cooled to 0°C, ethylenediamine (12.8 ml, 0.192 mol) was slowly added dropwise, and the temperature should be controlled not to exceed 5°C. The reaction was maintained at 0°C-10°C overnight, and then acetic acid was added to quench the reaction. pH was adjusted to 5-6, and the reaction mixture was purified using reverse-phase chromatography column (C18) (eluted with 10% - 60% acetonitrile/water gradient solution). Suitable eluate was collected and lyophilized to obtain compound 4 (2.5 g, 83.3%).

### Example 5: Preparation of compound 4 from compound 7

Compound 7 (3.0 g, 2.59 mmol) dissolved in mixture consisting of 6.5 ml water and 6.5 ml methanol was cooled to -10°C, ethylenediamine (12.8 ml, 0.192 mol) was slowly added dropwise, and the temperature should be controlled not to exceed 2°C. The reaction was maintained at 25°C for 6 hr, and then acetic acid was added to quench the reaction. pH was adjusted to 5-6, and the reaction mixture was purified using reverse-phase chromatography column (C18) (eluted with 10% - 60 % acetonitrile/water gradient solution). Suitable eluate was collected and lyophilized to obtain compound 4 (2.6 g, 86.6%).

### Example 6: Preparation of compound 1 from compound 4

Compound 4 (9.0 g, 8.22 mmol) and phenyl boric acid (1.103 g, 9.047 mmol) were dissolved in dried tetrahydrofuran (382.5 ml) under N₂ protection. The suspension was refluxed and dehydrated through 3A molecular sieve for 1.5 hr. Then, the reaction solution was clarified. Dried tetrahydrofuran was added to the original volume, the reaction was cooled to about 20°C, and BSTFA(7.28 ml, 27.1 mmol) was added dropwise. The reaction was maintained at this temperature for 1 hr;then, cooled to about -10°C. Borane solution (1.0 M , 24.67ml) in tetrahydrofuran was added dropwise, and the temperature should be controlled not to exceed 0°C. The reaction was warmed to 0-10°C and maintained for 3 hr. The reaction was cooled to below 0°C. 2 N HCl solution (22.62 ml) was slowly added dropwise, and the reaction was maintained at -5 to +5°C for 2.5 hr with agitation. The reaction mixture was purified using reverse-phase chromatography column (C18) (eluted with 10% - 60 % acetonitrile/water gradient solution). Suitable fractions were collected and lyophilized to obtain the crude product 4.0 g, which was crystallized to obtain compound 1 (3.6 g, 40%).
MS(ESI) 1093.6(M+H⁺)
¹H NMR (500 MHz, CD30D) 7.12 (m, 2H), 6.75 (m, 2H), 4.97 (d,1H), 4.91 (d, 1H), 4.66 (d, 1H), 4.60(dd, 1H), 4.56-4.51 (om, 2H), 4.48 (dd, 1H), 4.32-4.28 (om, 3H) 4.22 (dd, 1H), 4.18 (d, 1H), 4.08-3.96 (om, 3H), 3.83 (m, 1H), 3.76 (d, 1H), 3.05 (t, 2H), 3.02-2.76 (om, 4H), 2.41 (dd,1H), 2.29-2.17 (om, 3H) 2.11-1.78 (om, 5H), 1.90(s, 6H), 1.58 (m, 2H), 1.53-1.19 (om, 15H), 1.16 (d, 3H), 1.13-1.00 (om, 2H), 0.91 (m, 1H), 0.87 (t, 3H),
0.85 (degenerate d, 6H);
13C NMR (125 MHz, CD30D) 180.8, 176.7, 174.6, 174.1, 174.0, 173.3, 173.2, 169.4, 159.0, 116.7, 77.8, 76.1, 75.5, 72.5, 71.8, 70.6, 69.8, 68.9, 64.8, 63.3, 58.9, 58.8, 57.6, 56.7, 56.5, 51.7, 47.5, 46.4, 44.5, 40.9, 39.5, 38.9, 38.5, 37.4, 36.2, 35.1, 33.4, 31.7, 31.6, 31.4, 31.3, 31.1, 30.84, 30.81, 28.5, 27.6, 24.8

### Example 7: Preparation of compound 1 from compound 4

Compound 4 (9.0 g, 8.22 mmol) and phenyl boric acid (1.103 g, 9.047 mmol) were dissolved in dried tetrahydrofuran (382.5 ml) under N₂ protection. The suspensionwas refluxed and dehydrated through 3A molecular sieve for 1.5 hr. Then, the reaction solution was clarified. Dried tetrahydrofuran was added to the original volume, the reaction was cooled to about 20°C, and BSTFA(7.28 ml, 27.1 mmol) was added dropwise. Afterwards, the reaction was refluxed for 30 min, and then the reaction was cooled to about -10°C. Borane solution (1.0 M, 24.67 ml) in tetrahydrofuran was added dropwise, and the temperature should be controlled not to exceed 0°C. The reaction was warmed to 0-10°C and maintained for 3 hr. The reaction was cooled to below 0°C. 2 N HCl solution (22.62 ml) was slowly added dropwise and the reaction was maintained at -5-5°C for 2.5 hr with agitation. The reaction mixture was purified using reverse-phase chromatography column (C18) (eluted with 10% - 60 % acetonitrile/water gradient solution). Suitable fractions were collected and lyophilized to obtain the crude product 8.3 g, which was crystallized to obtain compound 1 (7.2 g, 80%).

While the preferred embodiments of the invention has been illustrated and described, it will be appreciated that after reading the description above, many variations and modifications without departing from the spirit and scope of the invention may be made by the skilled in the art, and these equivalents also fall within the scope as defined by the appended claims

## Claims

1. An azacyclohexapeptide represented by the following formula 4: 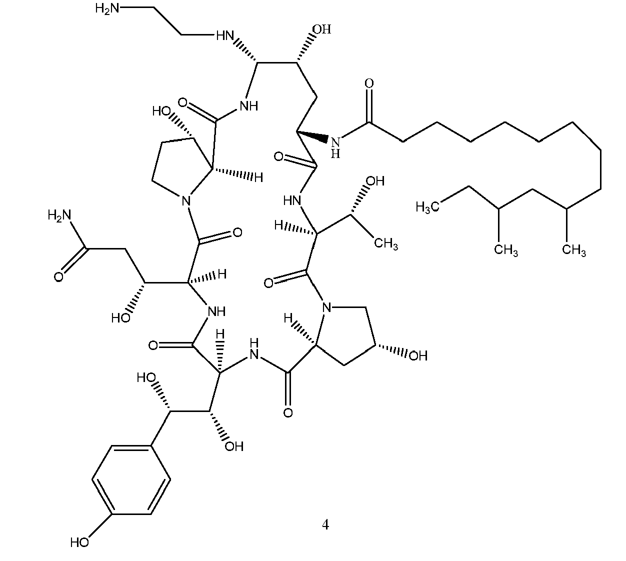 or pharmaceutically acceptable salts thereof.

2. A preparation method for the azacyclohexapeptide or pharmaceutically acceptable salts thereof according to claim 1, comprising the following steps:
( a ) mixing the compound of formula 2 with a compound containing strong leaving group to obtain the compound of formula 3; and
( b ) mixing the compound of formula 3 with ethylenediamine to obtain the azacyclohexapeptide or pharmaceutically acceptable salts thereof of claim 1; wherein the strong leaving group compound is the sulfhydryl-substituted aromatic ring compound represented by R-SH, wherein R is selected from phenyl, 4-methoxyphenyl, methylimidazolyl, or benzimidazolyl;

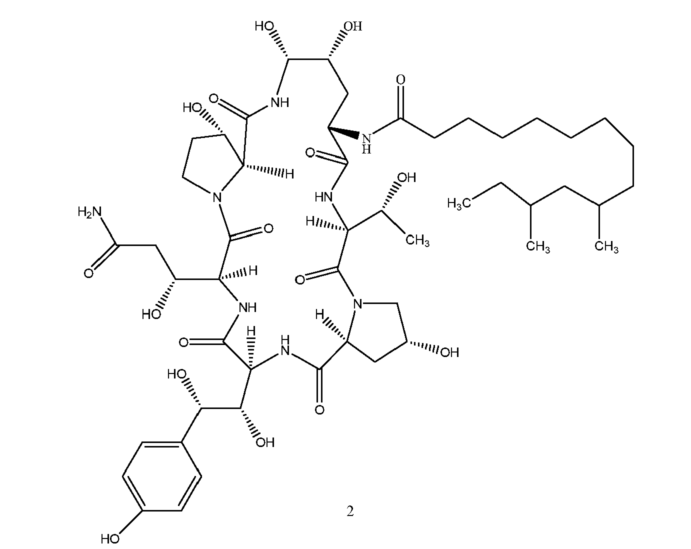

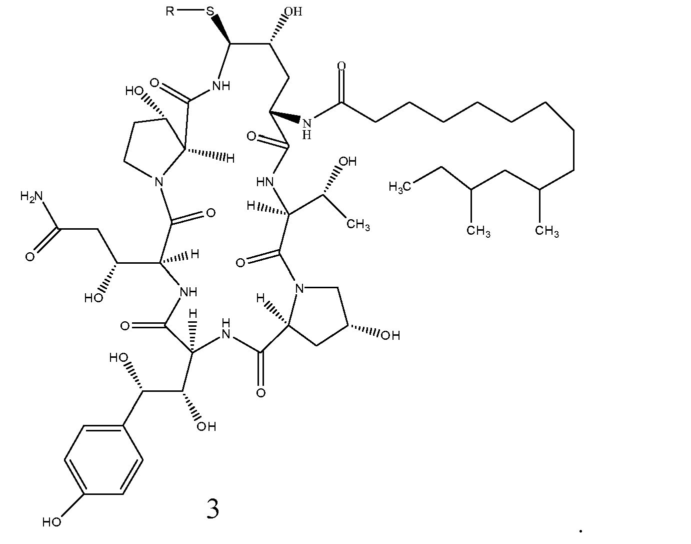

3. The preparation method according to claim 2, wherein step (a) comprises mixing the compound of formula 2 with the compound containing strong leaving group dissolved in an acid solution, wherein said acid is selected from p-toluene sulfonic acid, methane-sulfonic acid, camphorsulfonic acid, trifluoromethanesulfonic acid or trifluoroacetic acid.

4. The preparation method according to claim 2, wherein the temperature for performing the mixing in step (a) is in a range of -50°C to 40°C;preferably -15°C.

5. The preparation method according to claim 2, wherein step (b) comprisies mixing the compound of formula 3 with ethylenediamine dissolved in the following solvents: water, methanol, ethanol, aqueous alcohol, tetrahydrofuran, isopropanol, trifluoroethanol, acetonitrile, or dichloromethane.

6. The preparation method according to claim 5, wherein the aqueous alcohol is selected from aqueous methanol or aqueous ethanol.

7. The preparation method according to claim 2, wherein the temperature for performing the mixing in step (b) is in a range of -10°C to +40°C; preferably 25°C.

8. The use of the azacyclohexapeptide or pharmaceutically acceptable salts thereof according to claim 1 in the manufacture of a medicament for preventing or treating diseases caused by fungous infection.

9. The preparation method for the compound of formula 1, wherein the method comprises the following steps:
( i ) mixing the compound of formula 4 with hydroxyl-protecting agent to obtain the compound of formula 4 with hydroxy being protected; and
( ii ) mixing the compound of formula 4 with hydroxy being protected with borane complex to obtain the compound of formula 1; 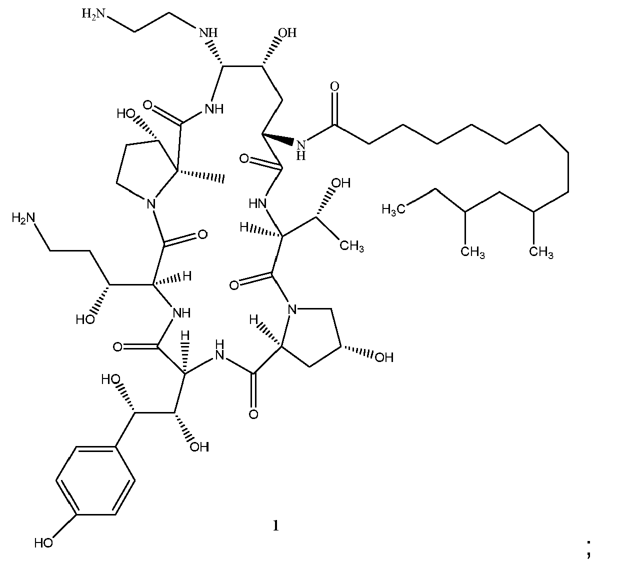 wherein the hydroxyl-protecting agent is selected from boric acid protective agents or silane agents;
wherein the borane complex is selected from the complex of methyl borane and tetrahydrofuran, methyl borane and dimethyl sulfide, methyl borane and dibenzyl sulfide, methyl borane and diphenyl sulfide, methyl borane and 1,4-oxathiane, or the complex of BH₂Cl and dimethyl sulfide; preferably, the complex of methyl borane and tetrahydrofuran or methyl borane and dimethyl sulfide.

10. The preparation method according to claim 9, wherein the temperature for performing the mixing in step ( ii ) is in a range of -20°C to +40°C; preferably 0°C-10°C.

11. The preparation method according to claim 9, wherein the method comprises the following steps:
( 1 ) mixing the compound of formula 2 with a compound containing strong leaving group to obtain the compound of formula 3;
( 2 ) mixing the compound of formula 3 with ethylenediamine to obtain the azacyclohexapeptide or pharmaceutically acceptable salts thereof of claim 1; and
( 3 ) mixing the compound of formula 4 with borane complex to obtain the compound of formula 1;
wherein the compound containing strong leaving group is the sulfhydryl-substituted aromatic ring compound represented by R-SH, wherein R is selected from phenyl, 4-methoxyphenyl, methylimidazolyl, or benzimidazolyl;
wherein the borane complex is selected from the complex of methyl borane and tetrahydrofuran, methyl borane and dimethyl sulfide, methyl borane and dibenzyl sulfide, methyl borane and diphenyl sulfide, methylborane and 1,4-oxathiane, or the complex of BH₂Cl and dimethyl sulfide.
